# EUROPEAN PATENT APPLICATION

(11) **EP 4 635 519 A1**
(43) Date of publication of application: **22.10.2025**
(21) Application number: 23902690.9
(22) Date of filing: 12.12.2023
(51) Int. Cl.: A61K 48/00, C12N 5/16, A61K 35/12, A01K 67/00

(54) **METHOD FOR GENE REGULATION AND USE THEREOF**

(30) Priority: 12.12.2022 CN 202211589385
(71) Applicant: Peking University, Beijing 100091 (CN)
(72) Inventor: HU, Xuefei, Beijing 100091 (CN); RAO, Yi, Beijing 100091 (CN)
(74) Representative: Murgitroyd & Company
(86) International application number: PCT/CN2023/138081
(87) International publication number: WO 2024/125494

(57) **Abstract**

Provided is a method for preparing a non-human animal with GIGYF1 gene deletion. The non-human animal can be used to screen the use of a recombinant nucleic acid in treating specific diseases. Specifically, also provided is use of the recombinant nucleic acid in treating metabolic diseases and/or symptoms.

## Description

### TECHNICAL FIELD

The present application relates to the field of biomedicine, and specifically to a method for gene regulation and use thereof.

### BACKGROUND

Type 2 diabetes (T2DM) affects the health of a large number of patients, and diabetes is also a major risk for coronary artery and peripheral artery disease and other complications. Although there are many drugs and other therapies, many oral T2DM drugs have poor therapeutic effects in subjects and have adverse effects.

Therefore, there is an urgent need in the art for a therapy that can be used for the alleviation, prevention and/or treatment of metabolic diseases.

### SUMMARY OF THE INVENTION

The present application provides a recombinant nucleic acid that can be used for the alleviation, prevention and/or treatment of metabolic diseases and/or symptoms.

In one aspect, the present application provides use of a formulation for interfering with the expression of GIGYF 1, GIGYF1 variants, truncated versions and analogs thereof in the preparation of a drug for alleviating, preventing and/or treating a specific disease and/or symptom.

In one aspect, the present application provides a formulation for interfering with the expression of GIGYF1, GIGYF1 variants, truncated versions and analogs thereof, and the formulation is used to alleviate, prevent and/or treat a specific disease and/or symptom.

In one aspect, the present application provides a method for alleviating, preventing and/or treating a specific disease and/or symptom, comprising interfering with the expression of GIGYF 1, GIGYF1 variants, truncated versions and analogs thereof.

In one aspect, the present application provides a vector comprising a nucleic acid molecule encoding GIGYF1, GIGYF1 variants, truncated versions and analogs thereof. In certain embodiments, the vector is an AAV vector. In certain embodiments, the vector is AAV9.

In another aspect, the present application provides a cell comprising a formulation interfering with GIGYF1, GIGYF1 variants, truncated versions and analogs thereof.

In another aspect, the present application provides a kit comprising a nucleic acid encoding GIGYF1, GIGYF1 variants, truncated versions and analogs thereof, a vector comprising the above nucleic acid and/or the cell of the present application.

In certain embodiments, the GIGYF1 comprises an amino acid sequence as set forth in SEQ ID NO: 2.

In certain embodiments, the GIGYF1 comprises an amino acid sequence as set forth in SEQ ID NO: 1. In certain embodiments, the amino acid sequence of GIGYF1 is as set forth in SEQ ID NO: 1.

In certain embodiments, the nucleotide sequence encoding the GIGYF1 protein is as set forth in SEQ ID NO: 5. In certain embodiments, the gene encoding the GIGYF1 protein comprises a nucleotide sequence as set forth in SEQ ID NO: 5.

In certain embodiments, the GIGYF1 is configured to be delivered to a tissue or cell selected from the following group: liver, heart, muscle, pancreas and brain.

In certain embodiments, the disease and/or symptom is associated with GIGYF1 expression.

In certain embodiments, the GIGYF1 is used to alleviate, prevent and/or treat a metabolic disease and/or symptom.

In certain embodiments, the GIGYF1 is used to alleviate, prevent and/or treat diabetes and/or a complication thereof.

In certain embodiments, the disease and/or symptom is selected from the following group: developmental delay, hyperglycemia, hyperlipidemia, increased ratio of glycosylated hemoglobin, insulin resistance, decreased glucose tolerance, increased fat, fatty liver, spleen hyperplasia, decreased insulin secretion, increased glucagon secretion, decreased reproductive capacity, kidney damage, skin damage, swollen finger (toe) joints, heart damage, and cognitive impairment.

After administering the proteins of GIGYF1, GIGYF1 variants, truncated versions and analogs thereof or nucleic acids encoding the proteins described in the present application, the phenotype and/or symptom of diabetes is ameliorated.

In another aspect, the present application provides a modified non-human animal, wherein the expression level of the GIGYF1 gene of the modified non-human animal is reduced compared with a wild-type non-human animal. In certain embodiments, the modified non-human animal has a reduced expression amount of GIGYF1 protein compared with a wild-type non-human animal. In certain embodiments, the GIGYF1 protein comprises an amino acid sequence as set forth in SEQ ID NO.1.

In certain embodiments, the GIGYF1 gene of the modified non-human animal is knocked out or knocked down. In certain embodiments, the GIGYF1 gene comprises a nucleic acid sequence as set forth in SEQ ID NO.5.

In certain embodiments, the modification includes contacting the genome of a stem cell or fertilized egg of a non-human animal cell with the following substances: 1) CRISPR-associated (Cas) protein; and 2) one or more ribonucleic acid (RNA) sequences. For example, the one or more ribonucleic acid (RNA) sequences comprise a sequence complementary to the GIGYF1 gene, and a sequence that binds to the Cas protein.

In certain embodiments, for the modification, the Cas protein is Cas9. In certain embodiments, the stem cell is selected from the following group: an embryonic stem cell, an adult stem cell, a totipotent stem cell, a unipotent stem cell, and an induced pluripotent stem cell.

In certain embodiments, the sequence that binds to the Cas protein comprises a tracrRNA sequence.

In certain embodiments, the one or more ribonucleic acid (RNA) sequences comprise a sequence as set forth in any one of SEQ ID NOs: 3-4.

In certain embodiments, the modified non-human animal exhibits a phenotype and/or symptom of a metabolic disease.

In certain embodiments, the modified non-human animal exhibits a phenotype and/or symptom of diabetes.

In certain embodiments, the phenotype and/or symptom of diabetes is selected from any one or more of the following groups: (1) developmental delay; (2) increased blood lipids; (3) increased blood glucose concentration; (4) abnormal fat metabolism, for example, increased fat mass in females, decreased fat mass in males, fatty liver, lipomas and spleen hyperplasia; (5) reproductive abnormalities, for example, decreased sperm count in male mice; (6) poor renal function, for example, abnormal urine color (yellow urine); (6) partial hair loss, skin ulcers or swollen finger (toe) joints; (7) increased mortality; (8) abnormal heart condition; (9) cognitive abnormalities; (10) slower glucose clearance; (11) reduced sensitivity to insulin; (12) increased expression of tumor necrosis factor α; (13) decreased glucagon secretion; (14) decreased insulin secretion and (15) decreased energy exchange.

In another aspect, the present application provides a living part of a modified non-human animal. In certain embodiments, the non-human animal includes a mouse.

In another aspect, the present application provides a cell, tissue or organ of a modified non-human animal. In certain embodiments, the non-human animal includes a mouse.

In another aspect, the present application provides use of the modified non-human animal in screening a drug for alleviating, preventing and/or treating a metabolic disease and/or symptom. In certain embodiments, the use includes administering one or more candidate agents to the modified non-human animal. If the metabolic disease and/or symptom of the modified non-human animal is ameliorated, then the one or more candidate agents are considered to be drugs that can alleviate, prevent and/or treat the metabolic disease and/or symptom; if the metabolic disease and/or symptom of the modified non-human animal is not ameliorated or worsened, then the one or more candidate agents are considered to be drugs that cannot alleviate, prevent and/or treat the metabolic disease and/or symptom.

In certain embodiments, the drug is used to alleviate, prevent and/or treat diabetes and/or a complication thereof.

In certain embodiments, the non-human animal includes a mouse.

In another aspect, the present application provides a method for screening a drug for relieving, preventing and/or treating a metabolic disease and/or symptom. If one or more candidate agents can reduce the expression level of the GIGYF1 gene or the expression amount of the GIGYF1 protein in the body, then the one or more candidate agents are identified as drugs that can relieve, prevent and/or treat a metabolic disease and/or symptom. If one or more candidate agents cannot reduce the expression level of the GIGYF 1 gene or the expression amount of the GIGYF 1 protein in the body, then the one or more candidate agents are identified as drugs that cannot relieve, prevent and/or treat a metabolic disease and/or symptom.

Those skilled in the art can easily discern other aspects and advantages of the present application from the following detailed description. Only exemplary embodiments of the present application are shown and described in the following detailed description. As those skilled in the art will realize, the contents of the present application enable those skilled in the art to make changes to the specific embodiments disclosed without departing from the spirit and scope of the invention covered by the present application. Accordingly, the drawings and descriptions in the specification of the present application are illustrative only and not restrictive.

### BRIEF DESCRIPTION OF DRAWINGS

The specific features of the invention to which the present application relates are set forth in the appended claims. The features and advantages of the invention to which the present application relates can be better understood by reference to the exemplary embodiments described in detail below and the drawings. The accompanying drawings are briefly described as follows:
FIG. 1 shows the random blood glucose concentration of the *Gigyf1* knockout mice.
FIG. 2 shows the fasting blood glucose concentration of the *Gigyf1* knockout mice.
FIG. 3 shows the results of the glucose tolerance test of the *Gigyf1* knockout mice.
FIG. 4 shows the results of the insulin sensitivity test of the *Gigyf1* knockout mice.

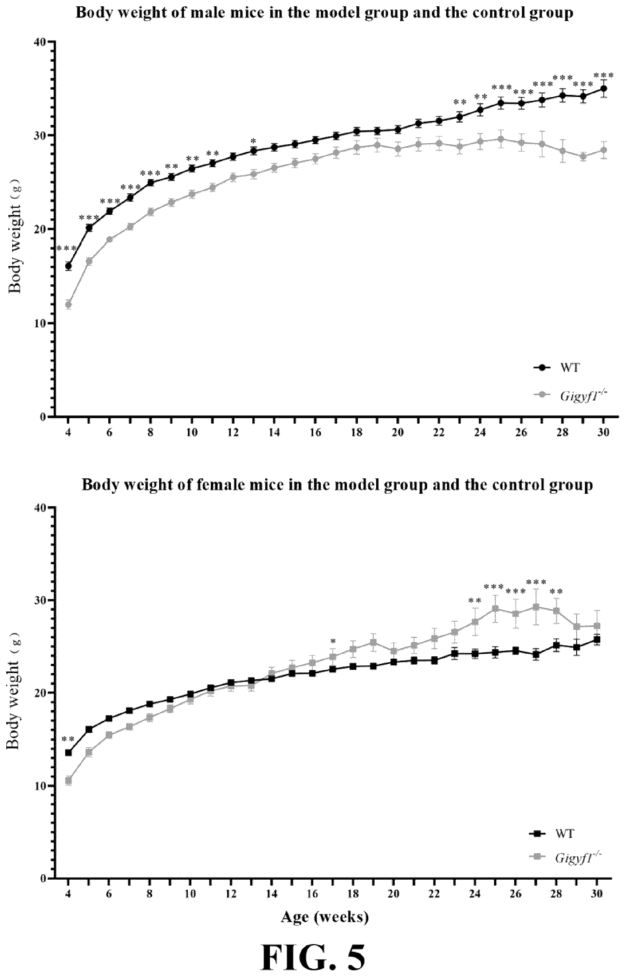

FIG. 5 shows the body weight of the *Gigyf1* knockout mice.
FIG. 6 shows the body fat percentage of the *Gigyf1* knockout mice.
FIG. 7 shows the energy expenditure of the *Gigyf1* knockout mice.
FIG. 8 shows the fasting insulin and glucagon levels of the *Gigyf1* knockout mice.
FIG. 9 shows the survival curve of the *Gigyf1* knockout mice.
FIG. 10 shows the response of the middle-aged knockout mice to glucose and insulin after delivery of *GIGYF1.*
FIG. 11 shows the response of the old knockout mice to glucose and insulin after delivery of *GIGYF1.*

### DETAILED DESCRIPTION

The embodiments of the invention of the present application will be described below with specific examples. Those skilled in the art may easily understand other advantages and effects of the invention of the present application from the disclosure of the specification.

### DEFINITION OF TERMS

In the present application, the terms "GRB10 Interacting GYF Protein 1" and "GIGYF1 protein" generally have the same meaning and are used interchangeably herein. GIGYF1 is a member of the GIGYF family, and the protein encoded by GIGYF1 contains a GYF motif. For example, the GIGYF1 may comprise unprocessed GIGYF1, any processed form of GIGYF1, a GIGYF1 variant, or a substance containing a functionally active fragment of GIGYF1. For more information about human GIGYF1 protein, see Uniport O75420; for more information about mouse GIGYF1 protein, see Uniport Q99MR1.

In the present application, the term "GIGYF1 gene" generally refers to a gene encoding a member of the gyf adaptor protein family, GIGYF1 protein. For more information about the human GIGYF1 gene, see Ensembl: ENSG00000146830 or NCBI Gene ID: 64599.

In the present application, the term "GIGYF1" or its various uppercase and lowercase forms may refer to proteins, genes or RNA. When not explicitly stated, those skilled in the art can clearly know which substance the term "GIGYF1" or its uppercase and lowercase forms represents based on the context. In the present application, the term "encode" generally refers to the inherent properties of a specific nucleotide sequence in a polynucleotide such as a gene, DNA or mRNA, that is, the sequence can serve as a template to synthesize other polymers and macromolecules with a specified nucleotide sequence (such as rRNA, tRNA and mRNA) or a specified amino acid sequence in a biological process, and the resulting biological properties. Therefore, when the transcription and translation of the mRNA corresponding to a gene in a cell or other biological system leads to the production of a protein, the gene, cDNA, or RNA can encode the protein. Both the coding strand (whose nucleotide sequence is the same as the mRNA sequence, usually provided in the sequence table) and the non-coding strand (used as a template for transcription of a gene or cDNA) can be referred to as encoding a protein or other product of the gene or cDNA.

In the present application, the term "vector" generally refers to an expression vector containing the recombinant nucleic acid or fusion nucleic acid DNA sequence of the present application and appropriate transcription/translation control signals. Methods for constructing vectors may include in vitro recombinant DNA technology, DNA synthesis technology, in vivo recombination technology, etc. The DNA sequence can be effectively linked to an appropriate promoter in the expression vector to guide mRNA synthesis. The expression vector may further include a ribosome binding site for translation initiation, a regulatory sequence, and a transcription terminator. The regulatory sequence may include a promoter, an enhancer, a transcription termination signal, a polyadenylation sequence, a replication origin, a nucleic acid restriction site, and a homologous recombination site operably linked to the nucleic acid sequence. The vector may further include a selective marker, for example, to determine the expression of the vector in a growth system (such as a bacterial cell) or in a pancreatic, liver, or muscle target cell. For example, a vector containing the above-mentioned appropriate DNA sequence and an appropriate promoter or regulatory sequence can be used to transform an appropriate host cell to enable the host cell to express a polypeptide.

In the present application, the term "viral vector" generally refers to a gene therapy vector suitable for transduction and expression in a target cell. For example, the target cell may be a pancreatic, liver, or muscle target cell. The viral vector includes those derived from adenovirus, adeno-associated virus (AAV), including mutated forms, retrovirus, lentivirus, herpes virus, vaccinia virus, MMLV, GaLV, simian immunodeficiency virus (SIV), HIV, poxvirus, and SV40. For example, the viral vector can generally remain in an extrachromosomal state without integrating into the genome of the pancreatic, liver, or muscle target cell, or can integrate into the genome of the pancreatic, liver, or muscle target cell. The viral vector can be an AAV vector, such as a self-complementary adeno-associated virus (scAAV). Selective targeting can be achieved using specific AAV serotypes (AAV serotype 2 to AAV serotype 12) or modified versions of any of these serotypes, including AAV 4YF and AAV 7m8 vectors.

In the present application, the term "adeno-associated virus" or "AAV" is also commonly referred to as adeno-associated virus, which belongs to the genus dependovirus in the family parvoviridae, is the simplest type of single-stranded DNA defective virus discovered so far and requires a helper virus (usually adenovirus) to participate in replication. Adeno-associated virus encodes the cap and rep genes in the two terminal inverted repeat sequences (ITR). The terminal inverted repeat sequences are crucial for viral replication and packaging. The cap gene encodes the viral capsid protein, and the rep gene is involved in viral replication and integration. Adeno-associated viruses can infect a variety of cells. For example, adeno-associated viruses can be used as recombinant adeno-associated virus vectors to integrate into the genome of the cells they infect in a stable and site-specific manner. Adeno-associated virus vectors can be prepared by standard methods in the art, and any serotype of adeno-associated virus is suitable. Replication-deficient recombinant adeno-associated virus can be prepared by co-transfecting the following plasmids into a cell line infected with a human helper virus (e.g., adenovirus): a plasmid containing the nucleic acid sequence of interest flanked by two adeno-associated virus inverted terminal repeat sequence (ITR) regions, and a plasmid carrying adeno-associated virus encapsidation genes (rep and cap genes). The resulting adeno-associated virus recombinant is then purified by standard techniques. For example, the recombinant adeno-associated virus vector can be encapsidated into a virus particle (e.g., an AAV viral particle including but not limited to AAV1, AAV2, AAV3, AAV4, AAV5, AAV6, AAV7, AAV8, AAV9, AAV10, AAV11, AAV12, AAV13, AAV14, AAV15, and AAV16).

In the present application, the term "serotype" generally refers to the detection of epitopes on the surface of the adeno-associated virus capsid by serological methods and the typing of the adeno-associated virus. Adeno-associated virus has a variety of common serotypes and more than 100 virus variants. In the present application, AAV capsid, ITR and other selected AAV components are selected from any AAV, including but not limited to AAV1, AAV2, AAV3, AAV4, AAV5, AAV6, AAV7, AAV8, AAV9, AAV8bp, AAV7M8 and AAVAnc80, any known or mentioned AAV variants or AAVs yet to be discovered or their variants or mixtures.

In the present application, the term "host cell" generally refers to a prokaryotic cell, or a lower eukaryotic cell, or a higher eukaryotic cell, such as a mammalian cell (including human and non-human mammals). Representative examples may comprise the following group: animal cells such as CHO, NS0, COS7, or 293 cells. For example, a neural cell is selected as the host cell. For example, the host cell is selected from the following group: a pancreas cell, a liver cell, a muscle cell, or a combination thereof. Transformation of the host cell with recombinant DNA can be performed using conventional techniques well known to those skilled in the art. When the host is a prokaryotic organism such as Escherichia coli, competent cells that can absorb DNA can be harvested after the exponential growth phase and treated with the CaCl₂ method, and the steps used are well known in the art. Another method is to use MgCl₂. If necessary, transformation can also be performed by electroporation. When the host is a eukaryotic organism, the following DNA transfection methods can be selected: calcium phosphate coprecipitation method, conventional mechanical methods such as microinjection, electroporation, liposome packaging, etc.

In the present application, the term "culture" can generally be culturing host cells by conventional methods to express the protein encoded by the gene of the present application. Depending on the host cell used, the culture medium used in the culture can be selected from various conventional culture media. The culture is carried out under conditions suitable for the growth of the host cell. When the host cells grow to an appropriate cell density, the selected promoter is induced by a suitable method (such as temperature conversion or chemical induction), and then the cells are further cultured for a period of time.

In the present application, the term "recombinant nucleic acid" generally refers to a recombinant gene sequence. The recombinant nucleic acid of the present application may be in the form of DNA or RNA. For example, the recombinant nucleic acid is DNA. The DNA form includes cDNA, genomic DNA or artificially synthesized DNA, DNA may be single-stranded or double-stranded, and DNA may be a coding strand or a non-coding strand.

In the present application, the term "operably linked" may mean that a nucleic acid sequence is functionally associated with a sequence to which it is operably linked, in such a way that they affect the expression or function of each other. For example, a nucleic acid sequence operably linked to a promoter will have an expression pattern affected by the promoter.

In the present application, the term "expression" may refer to the recombinant nucleic acid, fusion nucleic acid, or vector of the present application transcribing the RNA of interest and/or translating the protein of interest in the host cell.

In the present application, the term "treat" generally refers to intervention that attempts to alter the natural course of the subject being treated, and can be performed either prophylactically or during the course of clinical pathology. The desired effects include, but are not limited to, preventing the occurrence or recurrence of the disease, alleviating the symptom, curbing, reducing or inhibiting any direct or indirect pathological consequences of the disease, ameliorating or alleviating the disease state, and causing remission or improving prognosis.

In the present application, the term "pharmaceutically acceptable pharmaceutical carrier" or "excipient" refers to one or more compatible solid or liquid fillers or gel substances, which are suitable for human use and must have sufficient purity and sufficiently low toxicity. Compatibility herein refers to the ability of the components in the composition to blend with the active ingredients of the present application and with each other without significantly reducing the efficacy of the active ingredients. Some examples of pharmaceutically acceptable carriers include cellulose and derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween), wetting agents (such as sodium lauryl sulfate), colorants, flavorings, stabilizers, antioxidants, preservatives, pyrogen-free water, etc.

In the present application, the terms "subject", "object in need" generally refer to any mammal or non-mammal. Mammals include, but are not limited to, humans, vertebrates such as rodents, non-human primates, rabbits, rats, mice, horses, dogs, cats, pigs, sheep, and goats.

In the present application, the term "about" generally refers to a value or composition within an acceptable error range of a particular value or composition determined by those of ordinary skill in the art, which will depend in part on how the value or composition is measured or determined. For example, "about 100" includes 99 and 101 and all values in between (e.g., 99.1, 99.2, 99.3, 99.4, etc.).

In the present application, the term "containing" or "including (comprising)" generally refers to open, semi-closed and closed. In other words, the term also includes "essentially consisting of..." or "consisting of..."

In the present application, sequence "identity" or "sameness" is usually determined by comparing two aligned sequences along a predetermined comparison window (which may be 50%, 60%, 70%, 80%, 90%, 95% or 100% of the length of the reference nucleotide sequence or protein) and determining the number of positions where identical residues occur. Typically, this is expressed as a percentage. The measurement of sequence "identity" or "sameness" of nucleotide sequences is a method well known to those skilled in the art. For example, "identity" can refer to the correlation between two sequences based on nucleotide-by-nucleotide comparison within a specific comparison window or segment. Thus, identity is defined as the degree of sameness, correspondence or equivalence between two DNA segments on the same strand (i.e., either the sense strand or antisense strand). "Percent sequence identity" is calculated as follows: two optimally aligned sequences are compared within a specific region; the number of positions where the identical bases or amino acids occur in the two sequences is determined to obtain the number of matching positions; the number of such positions is divided by the total number of positions in the compared segment, and the quotient is multiplied by 100. The optimal alignment of sequences can be performed by existing algorithms.

### DETAILED DESCRIPTION OF THE PRESENT INVENTION

The present application provides a recombinant nucleic acid that can be used for the alleviation, prevention and/or treatment of metabolic diseases and/or symptoms. The association between the GIGYF1 target and the disease has not yet been clearly elucidated; the present application provides a method and use for intervening in related diseases by interfering with GIGYF1. Preferably, the applicant has found an unexpected effect of intervening in diseases by interfering with the expression of GIGYF1 in specific tissues.

In one aspect, the present application provides use of a formulation for interfering with the expression of GIGYF1, GIGYF1 variants, truncated versions and analogs thereof in the preparation of a drug, which can be used to alleviate, prevent and/or treat a specific disease and/or symptom.

In one aspect, the present application provides a formulation for interfering with the expression of GIGYF1, GIGYF1 variants, truncated versions and analogs thereof, and the formulation can be used to alleviate, prevent and/or treat a specific disease and/or symptom.

In one aspect, the present application provides a method for alleviating, preventing and/or treating a specific disease and/or symptom, and the method can comprises interfering with the expression of GIGYF1, GIGYF1 variants, truncated versions and analogs thereof.

For example, the GIGYF1 comprises a GYF motif, and the sequence of the GYF motif is as set forth in SEQ ID NO: 2. For example, the GIGYF1 of the present application may comprise a functionally active fragment of GIGYF1, such as a truncated version having the functionally active fragment that can maintain the biological activity of the GYF motif.

For example, the GIGYF1 comprises the expression product of 1 or more exons of GIGYF1. For example, the GIGYF1 may comprise the expression products of 27 exons of GIGYF1. For example, the GIGYF1 may comprise the expression products of 1, 2, 3, 4, 5, 6, 7, 8, 9, 10, 15, 20, 25, or 27 exons of GIGYF1.

For example, the GIGYF1 comprises human GIGYF1. For example, the GIGYF1 comprises mammalian GIGYF1. For example, the GIGYF1 comprises primate GIGYF1. For example, the GIGYF1 comprises human, monkey, rat, mouse, rabbit GIGYF1.

For example, the amino acid sequence of the GIGYF1 is as set forth in SEQ ID NO: 1. For example, the amino acid sequence of the GIGYF1 has about at least 50% identity with that of SEQ ID NO: 1. For example, the amino acid sequence of the GIGYF1 may have about at least 50%, about at least 60%, about at least 70%, about at least 80%, about at least 85%, about at least 90%, about at least 91%, about at least 92%, about at least 93%, about at least 94%, about at least 95%, about at least 96%, about at least 97%, about at least 98%, or about at least 99% identity with that of SEQ ID NO: 1.

For example, the nucleotide sequence encoding the GIGYF1 is as set forth in SEQ ID NO: 4. For example, the nucleotide sequence encoding the GIGYF1 has about at least 50% identity with that of SEQ ID NO: 4. For example, the nucleotide sequence encoding the GIGYF1 may have about at least 50%, about at least 60%, about at least 70%, about at least 80%, about at least 85%, about at least 90%, about at least 91%, about at least 92%, about at least 93%, about at least 94%, about at least 95%, about at least 96%, about at least 97%, about at least 98%, or about at least 99% identity with that of SEQ ID NO: 4.

For example, the formation for interfering with the expression of GIGYF1, GIGYF1 variants, truncated versions and analogs thereof may also optionally comprise a substance that reduces the expression of the gene. For example, the formation for interfering with the expression of GIGYF1, GIGYF1 variants, truncated versions and analogs thereof may comprise a CRISPR editing system, a small inhibitory RNA (siRNA), a microRNA (miRNA) or a small hairpin RNA (shRNA).

For example, the formation for interfering with the expression of GIGYF1, GIGYF1 variants, truncated versions and analogs thereof may also optionally comprise a substance that increases the expression of the gene. For example, the nucleic acid or nucleic acid sequence may be an oligonucleotide, a nucleotide, a polynucleotide; for example, the nucleic acid or nucleic acid sequence may be a fragment of any of the above; for example, the nucleic acid or nucleic acid sequence may be a DNA or RNA of genomic or synthetic origin, which may be single-stranded or double-stranded, and may represent a sense strand or an antisense strand; for example, the nucleic acid or nucleic acid sequence may be a peptide nucleic acid (PNA); or for example, the nucleic acid or nucleic acid sequence may be any DNA-like or RNA-like substance of natural or synthetic origin. For example, the nucleic acids of the present application may include oligonucleotides, nucleotides, polynucleotides, or any fragment of any of these; for example, the nucleic acids of the present application may include DNA or RNA of genomic or synthetic origin (e.g., mRNA, rRNA, tRNA, and iRNA), which may be single-stranded or double-stranded; for example, the nucleic acids of the present application may include sense strands or antisense strands, or peptide nucleic acids (PNA), or any DNA-like or RNA-like substance of natural or synthetic origin, including, for example, iRNA, ribonucleoproteins (e.g., for example, double-stranded iRNA, e.g., iRNP). For example, nucleic acids of the present application may include nucleic acids containing known analogs of natural nucleotides, i.e., modified oligonucleotides.

For example, the nucleic acid sequence of the GIGYF1 is packaged by non-viral vectors and/or viral vectors. For example, wherein the viral vectors may be selected from the following group: recombinant parvovirus, recombinant lentivirus, recombinant retrovirus and recombinant adenovirus. For example, wherein the non-viral vectors may be selected from the following group: naked DNA, naked RNA, chemically modified DNA, chemically modified RNA, inorganic particle vectors, lipid particle vectors, liposome-based vectors, polymer-based vectors or chitosan-based vectors.

For example, the vectors of the present application also include plasmids, expression vectors, recombinant viruses, any form of recombinant "naked DNA" vectors, etc. For example, the vectors of the present application may comprise nucleic acids that can infect, transfect, transiently or permanently transduce cells. For example, the vectors of the present application may be naked nucleic acids or nucleic acids complexed with proteins or lipids. For example, the vectors of the present application may comprise viral or bacterial nucleic acids and/or proteins and/or membranes (e.g., cell membranes, viral lipid envelopes, etc.). For example, the vectors of the present application may include but are not limited to replicons (e.g., RNA replicons, bacteriophages), and DNA fragments may be connected to the replicons and replicated. For example, the vectors of the present application may include but are not limited to RNA, autonomous self-replicating circular or linear DNA or RNA.

For example, the viral vectors comprise lentiviral vectors, retroviral vectors, adenoviral vectors and/or adeno-associated viral vectors. For example, the nucleic acid sequence of the GIGYF1 is packaged by a viral vector selected from the following group: AAV1, AAV2, AAV3, AAV4, AAV5, AAV6, AAV7, AAV8, AAVrh8, AAVrh8R, AAV9, AAV10, AAVrh10, AAV11, AAV12, AAV2R471A, AAV2/2-7m8, AAV DJ, AAV2 N587A, AAV2 E548A, AAV2 N708A, AAVV708K, sheep AAV, bovine AAV, mouse AAV, AAV1/AAV2 chimera, and rAAV2/HBoV1 serotype. For example, the viral vector can be AAV9.

For example, the GIGYF1 can be operably linked to a regulatory element. For example, the regulatory element may be selected from the following group: a promoter, a polyadenylation sequence, an intron, an enhancer, and a polyA sequence. For example, the promoter may comprise a liver-related specific promoter, a pancreas-related specific promoter, and/or a muscle-related specific promoter.

For example, the "promoter" of the present application may include all sequences that can drive the transcription of a coding sequence in a cell (e.g., a mammalian cell, such as a heart, lung, muscle, nerve or brain cell). For example, the "promoter" of the present application may include a cis-acting transcription control element and regulatory sequence that are involved in regulating or modulating the timing and/or rate of gene transcription. For example, the "promoter" of the present application may include a cis-acting transcription control element, such as an enhancer, a promoter, a transcription terminator, a replication origin, a chromosomal integration sequence, and a 5' and 3' untranslated region or intron sequence, which are involved in the regulation of transcription. For example, cis-acting sequences can interact with proteins or other biological molecules to implement (turn on/off, regulate, control, etc.) transcription.

For example, the present application may comprise "constitutive" promoters, which may be those that continuously drive expression under most environmental conditions and developmental states or cell differentiation states. For example, the present application may comprise "inducible" or "regulatable" promoters, which can direct the expression of the nucleic acid of the present application under the influence of environmental conditions, applied chemicals or developmental conditions.

For example, the GIGYF1 is configured to specifically target a tissue or cell. For example, the GIGYF1 is configured to target a tissue or cell that requires gene editing. For example, the GIGYF1 is configured to be delivered to a tissue or cell selected from the following group: liver, heart, muscle, pancreas and brain. For example, the GIGYF1 is configured to be delivered to a liver cell, a heart cell, a pancreatic cell, a brain cell, a nerve cell, and/or a reproductive cell. For example, the subject is female. For example, the subject is male.

For example, the formulation of the present application can be delivered by intramuscular (IM) injection, intravenous (IV) injection, subcutaneous injection, inhalation, biolistic particle delivery system (e.g., so-called "gene gun"), etc.

For example, the disease and/or symptom is associated with GIGYF1 expression. For example, the GIGYF1 is used to alleviate, prevent and/or treat a metabolic disease and/or condition. For example, the GIGYF1 is used to alleviate, prevent and/or treat diabetes and/or a complication thereof. For example, the disease and/or symptom is selected from the following group: developmental delay, hyperglycemia, hyperlipidemia, increased ratio of glycosylated hemoglobin, insulin resistance, decreased glucose tolerance, increased fat, fatty liver, spleen hyperplasia, decreased insulin secretion, increased glucagon secretion, decreased reproductive capacity, kidney damage, skin damage, swollen finger (toe) joints, heart damage, and cognitive impairment.

For example, the method of the present application can be used to ameliorate or treat any of a variety of diabetes-related heart diseases and cardiovascular diseases, for example, diabetes-related heart diseases and cardiovascular diseases, such as coronary artery disease; atherosclerosis; thrombosis; restenosis; vasculitis, including autoimmune vasculitis and viral vasculitis, such as polyarteritis nodosa, allergic granulomatous vasculitis, and rickettsial vasculitis; atherosclerotic aneurysm; myocardial hypertrophy; congenital heart diseases; ischemic heart diseases and angina; acquired valvular diseases/endocardial diseases; primary cardiomyopathy, including myocarditis; arrhythmia; and transplant rejection; metabolic cardiomyopathy and myocardial lesions, such as congestive cardiomyopathy, hypertrophic cardiomyopathy or restrictive cardiomyopathy; and/or heart transplantation. For example, the method of the present application can be used to treat, ameliorate or prevent (protect against) diabetes or prediabetes in a patient or individual; or inhibit weight gain in a patient or individual, or inhibit appetite, or stimulate or initiate weight loss; or treat, ameliorate or prevent (protect against) diabetes in a patient or individual.

In one aspect, the present application provides a cell comprising a formulation interfering with GIGYF1, GIGYF1 variants, truncated versions and analogs thereof. For example, the cell of the present application can be used to screen a candidate formulation that can interfere with GIGYF 1, GIGYF1 variants, truncated versions and analogs thereof. For example, the cell in which GIGYF 1, GIGYF1 variants, truncated versions and analogs thereof are interfered can be used for administration into a subject. For example, the cell can comprise a desired cell type, such as a mammalian cell. For example, the cell can contain a skeletal muscle cell or tissue, a heart cell, a liver cell, a spleen cell, a kidney cell, a lung cell, a pancreatic islet cell, a bone cell, a germ cell, a neural cell, etc.

For example, the present application provides products comprising the cells of the present application (e.g., cells modified to express GIGYF1-encoding polypeptides to implement the method of the present application) and use of cells prepared by the method of the present application. The products include, for example, in vivo implanted cells, implants, artificial organs, bioreactor systems, cell culture systems, cell culture plates, cell culture vessels, cell culture tubes, cell culture bottles and cell culture flasks, wherein the above products comprise cells modified to express GIGYF 1-encoding polypeptides to implement the method of the present application.

In one aspect, the present application provides a kit comprising a nucleic acid encoding GIGYF1, GIGYF1 variants, truncated versions and analogs thereof, a vector comprising the above nucleic acid and/or the cell of the present application. For example, the kit may further comprise instructions for recording methods, such as in vitro or ex vivo methods for treating, preventing or ameliorating diabetes or diabetic complications.

In another aspect, the present application provides a modified non-human animal, wherein the expression level of the GIGYF1 gene of the modified non-human animal is reduced compared with a wild-type non-human animal. For example, the expression level is reduced by at least 10%. For example, the expression level is reduced by at least 20%. For example, the expression level is reduced by at least 30%. For example, the expression level is reduced by at least 40%. For example, the expression level is reduced by at least 50%. For example, the expression level is reduced by at least 60%. For example, the expression level is reduced by at least 70%. For example, the expression level is reduced by at least 80%. For example, the expression level is reduced by at least 90%. For example, the expression level is reduced by at least 95%. For example, the expression level is reduced by at least 99%. For example, the expression level is reduced by at least 100%.

In certain embodiments, the modified non-human animal has a reduced expression amount of GIGYF1 protein compared with a wild-type non-human animal. For example, the expression level is reduced by at least 10%. For example, the expression level is reduced by at least 20%. For example, the expression level is reduced by at least 30%. For example, the expression level is reduced by at least 40%. For example, the expression level is reduced by at least 50%. For example, the expression level is reduced by at least 60%. For example, the expression level is reduced by at least 70%. For example, the expression level is reduced by at least 80%. For example, the expression level is reduced by at least 90%. For example, the expression level is reduced by at least 95%. For example, the expression level is reduced by at least 100%.

In certain embodiments, the *Gigyf1* protein comprises an amino acid sequence as set forth in SEQ ID NO.1. In certain embodiments, the *Gigyf1* protein comprises an amino acid sequence as set forth in SEQ ID NO. 1 or a functional fragment thereof.

In certain embodiments, the GIGYF1 gene of the modified non-human animal is knocked out or knocked down. In certain embodiments, the GIGYF1 gene comprises a nucleic acid sequence as set forth in SEQ ID NO.5.

For example, zinc-finger nuclease (ZFN) can be used to knock out or knock down the GIGYF1 gene of the modified non-human animal. For example, transcription activator-like effector nuclease (TALEN) can be used to knock out or knock down the GIGYF1 gene of the modified non-human animal. For example, CRISPR/Cas can be used to knock out or knock down the GIGYF1 gene of the modified non-human animal. For example, RNAi can be used to knock out or knock down the GIGYF1 gene of the modified non-human animal. For example, shRNA can be used to knock out or knock down the GIGYF1 gene of the modified non-human animal. For example, siRNA can be used to knock out or knock down the GIGYF1 gene of the modified non-human animal.

In certain embodiments, the modification includes contacting the genome of a stem cell or fertilized egg of a non-human animal cell with the following substances: 1) CRISPR-associated (Cas) protein; and 2) one or more ribonucleic acid (RNA) sequences. For example, the one or more ribonucleic acid (RNA) sequences comprise a sequence complementary to the GIGYF1 gene, and a sequence that binds to the Cas protein.

In certain embodiments, for the modification, the Cas protein is Cas9.

In certain embodiments, the stem cell is selected from the following group: an embryonic stem cell, an adult stem cell, a totipotent stem cell, a unipotent stem cell, and an induced pluripotent stem cell.

In certain embodiments, the sequence that binds to the Cas protein comprises a tracrRNA sequence.

In certain embodiments, the one or more ribonucleic acid (RNA) sequences comprise a sequence as set forth in any one of SEQ ID NOs: 3-4.

In certain embodiments, the modified non-human animal exhibits a phenotype and/or symptom of a metabolic disease.

In certain embodiments, the modified non-human animal exhibits a phenotype and/or symptom of diabetes.

In certain embodiments, the phenotype and/or symptom of the diabetes is selected from any one or more of the following groups: (1) developmental delay; (2) increased blood lipids; (3) increased blood glucose concentration; (4) abnormal fat metabolism, for example, increased fat mass in females, decreased fat mass in males, fatty liver, lipomas and spleen hyperplasia; (5) reproductive abnormalities, for example, decreased sperm count in male mice; (6) poor renal function, for example, abnormal urine color (yellow urine); (6) partial hair loss, skin ulcers or swollen finger (toe) joints; (7) increased mortality; (8) abnormal heart condition; (9) cognitive abnormalities; (10) slower glucose clearance; (11) reduced sensitivity to insulin; (12) increased expression of tumor necrosis factor α; (13) decreased glucagon secretion; (14) decreased insulin secretion and (15) decreased energy exchange.

In certain embodiments, the modified non-human animal has delayed development compared with a wild-type or unmodified non-human animal. For example, the development is delayed by at least 10%. For example, the development is delayed by at least 20%. For example, the development is delayed by at least 30%. For example, the development is delayed by at least 40%. For example, the development is delayed by at least 50%. For example, the development is delayed by at least 60%. For example, the development is delayed by at least 70%. For example, the development is delayed by at least 80%. For example, the development is delayed by at least 90%.

In certain embodiments, the modified non-human animal has increased blood glucose compared with a wild-type or unmodified non-human animal, and the blood glucose may include random blood glucose and/or fasting blood glucose. For example, the blood glucose is increased by at least 5%. For example, the blood glucose is increased by at least 10%. For example, the blood glucose is increased by at least 15%. For example, the blood glucose is increased by at least 20%. For example, the blood glucose is increased by at least 30%. For example, the blood glucose is increased by at least 40%. For example, the blood glucose is increased by at least 50%. For example, the blood glucose is increased by at least 60%. For example, the blood glucose is increased by at least 70%. For example, the blood glucose is increased by at least 80%. For example, the blood glucose is increased by at least 90%.

In certain embodiments, the modified non-human animal has increased body weight compared with a wild-type or unmodified non-human animal. For example, the body weight is increased by at least 5%. For example, the body weight is increased by at least 10%. For example, the body weight is increased by at least 15%. For example, the body weight is increased by at least 20%. For example, the body weight is increased by at least 30%. For example, the body weight is increased by at least 40%. For example, the body weight is increased by at least 50%. For example, the body weight is increased by at least 60%. For example, the body weight is increased by at least 70%. For example, the body weight is increased by at least 80%. For example, the body weight is increased by at least 90%.

In certain embodiments, the modified non-human animal has increased body fat percentage compared with a wild-type or unmodified non-human animal. For example, the body fat percentage is increased by at least 5%. For example, the body fat percentage is increased by at least 10%. For example, the body fat percentage is increased by at least 15%. For example, the body fat percentage is increased by at least 20%. For example, the body fat percentage is increased by at least 30%. For example, the body fat percentage is increased by at least 40%. For example, the body fat percentage is increased by at least 50%. For example, the body fat percentage is increased by at least 60%. For example, the body fat percentage is increased by at least 70%. For example, the body fat percentage is increased by at least 80%. For example, the body fat percentage is increased by at least 90%.

In certain embodiments, the modified non-human animal has reduced lean meat percentage compared with a wild-type or unmodified non-human animal. For example, the lean meat percentage is reduced by at least 5%. For example, the lean meat percentage is reduced by at least 10%. For example, the lean meat percentage is reduced by at least 15%. For example, the lean meat percentage is reduced by at least 20%. For example, the lean meat percentage is reduced by at least 30%. For example, the lean meat percentage is reduced by at least 40%. For example, the lean meat percentage is reduced by at least 50%. For example, the lean meat percentage is reduced by at least 60%. For example, the lean meat percentage is reduced by at least 70%. For example, the lean meat percentage is reduced by at least 80%. For example, the lean meat percentage is reduced by at least 90%.

In certain embodiments, the modified non-human animal has reduced energy expenditure compared with a wild-type or unmodified non-human animal. For example, the energy expenditure is reduced by at least 5%. For example, the energy expenditure is reduced by at least 10%. For example, the energy expenditure is reduced by at least 15%. For example, the energy expenditure is reduced by at least 20%. For example, the energy expenditure is reduced by at least 30%. For example, the energy expenditure is reduced by at least 40%. For example, the energy expenditure is reduced by at least 50%. For example, the energy expenditure is reduced by at least 60%. For example, the energy expenditure is reduced by at least 70%. For example, the energy expenditure is reduced by at least 80%. For example, the energy expenditure is reduced by at least 90%.

In certain embodiments, the modified non-human animal has reduced insulin secretion compared with a wild-type or unmodified non-human animal. For example, the insulin secretion is reduced by at least 5%. For example, the insulin secretion is reduced by at least 10%. For example, the insulin secretion is reduced by at least 15%. For example, the insulin secretion is reduced by at least 20%. For example, the insulin secretion is reduced by at least 30%. For example, the insulin secretion is reduced by at least 40%. For example, the insulin secretion is reduced by at least 50%. For example, the insulin secretion is reduced by at least 60%. For example, the insulin secretion is reduced by at least 70%. For example, the insulin secretion is reduced by at least 80%. For example, the insulin secretion is reduced by at least 90%.

In certain embodiments, the modified non-human animal has reduced glucagon secretion compared with a wild-type or unmodified non-human animal. For example, the glucagon secretion is reduced by at least 5%. For example, the glucagon secretion is reduced by at least 10%. For example, the glucagon secretion is reduced by at least 15%. For example, the glucagon secretion is reduced by at least 20%. For example, the glucagon secretion is reduced by at least 30%. For example, the glucagon secretion is reduced by at least 40%. For example, the glucagon secretion is reduced by at least 50%. For example, the glucagon secretion is reduced by at least 60%. For example, the glucagon secretion is reduced by at least 70%. For example, the glucagon secretion is reduced by at least 80%. For example, the glucagon secretion is reduced by at least 90%.

In certain embodiments, the modified non-human animal has increased expression of C-peptide compared with a wild-type or unmodified non-human animal. For example, the expression of C-peptide is increased by at least 5%. For example, the expression of C-peptide is increased by at least 10%. For example, the expression of C-peptide is increased by at least 15%. For example, the expression of C-peptide is increased by at least 20%. For example, the expression of C-peptide is increased by at least 30%. For example, the expression of C-peptide is increased by at least 40%. For example, the expression of C-peptide is increased by at least 50%. For example, the expression of C-peptide is increased by at least 60%. For example, the expression of C-peptide is increased by at least 70%. For example, the expression of C-peptide is increased by at least 80%. For example, the expression of C-peptide is increased by at least 90%.

In certain embodiments, the modified non-human animal has increased expression of tumor necrosis factor compared with a wild-type or unmodified non-human animal. For example, the expression of tumor necrosis factor is increased by at least 5%. For example, the expression of tumor necrosis factor is increased by at least 10%. For example, the expression of tumor necrosis factor is increased by at least 15%. For example, the expression of tumor necrosis factor is increased by at least 20%. For example, the expression of tumor necrosis factor is increased by at least 30%. For example, the expression of tumor necrosis factor is increased by at least 40%. For example, the expression of tumor necrosis factor is increased by at least 50%. For example, the expression of tumor necrosis factor is increased by at least 60%. For example, the expression of tumor necrosis factor is increased by at least 70%. For example, the expression of tumor necrosis factor is increased by at least 80%. For example, the expression of tumor necrosis factor is increased by at least 90%.

In another aspect, the present application provides use of the modified non-human animal in screening a drug for alleviating, preventing and/or treating a metabolic disease and/or symptom. In certain embodiments, the use includes administering one or more candidate agents to the modified non-human animal. If the metabolic disease and/or symptom of the modified non-human animal is ameliorated, then the one or more candidate agents are considered to be drugs that can alleviate, prevent and/or treat the metabolic disease and/or symptom; if the metabolic disease and/or symptom of the modified non-human animal is not ameliorated or worsened, then the one or more candidate agents are considered to be drugs that cannot alleviate, prevent and/or treat the metabolic disease and/or symptom.

In certain embodiments, the drug is used to alleviate, prevent and/or treat diabetes and/or a complication thereof.

### TECHNICAL SOLUTIONS

1. Use of a formulation for interfering with GIGYF1, GIGYF1 variants, truncated versions and analogs thereof in the preparation of a drug for alleviating, preventing and/or treating a specific disease and/or symptom.
2. The use according to technical solution 1, wherein the GIGYF1 comprises a GYF motif, and the sequence of the GYF motif is as set forth in SEQ ID NO: 2.
3. The use according to any one of technical solutions 1-2, wherein the GIGYF1 comprises the expression product of one or more exons of GIGYF1.
4. The use according to any one of technical solutions 1-3, wherein the GIGYF1 comprises human GIGYF1.
5. The use according to any one of technical solutions 1-4, wherein the amino acid sequence of the GIGYF1 is as set forth in SEQ ID NO: 1.
6. The use according to any one of technical solutions 1-5, wherein the nucleotide sequence encoding the GIGYF1 is as set forth in SEQ ID NO: 4.
7. The use according to any one of technical solutions 1-6, wherein the nucleic acid sequence of the GIGYF1 is packaged by a non-viral vector and/or a viral vector.
8. The use according to technical solution 7, wherein the viral vector comprises a lentiviral vector, a retroviral vector, an adenoviral vector and/or an adeno-associated viral vector.
9. The use according to any one of technical solutions 1-8, wherein the nucleic acid sequence encoding the GIGYF1 is packaged by a viral vector selected from the following group: AAV1, AAV2, AAV3, AAV4, AAV5, AAV6, AAV7, AAV8, AAVrh8, AAVrh8R, AAV9, AAV10, AAVrh10, AAV11, AAV12, AAV2R471A, AAV2/2-7m8, AAV DJ, AAV2 N587A, AAV2 E548A, AAV2 N708A, AAVV708K, sheep AAV, bovine AAV, mouse AAV, AAV1/AAV2 chimera, and rAAV2/HBoV1 serotype.
10. The use according to any one of technical solutions 1-9, wherein the nucleic acid sequence encoding the GIGYF1 is operably linked to a regulatory element.
11. The use according to technical solution 10, wherein the regulatory element is selected from the following group: a promoter, a polyadenylation sequence, an intron, an enhancer, and a polyA sequence.
12. The use according to technical solution 11, wherein the promoter may comprise a liver-related specific promoter, a pancreas-related specific promoter, and/or a muscle-related specific promoter.
13. The use according to any one of technical solutions 1-12, the GIGYF1 is configured to specifically target a tissue or cell.
14. The use according to any one of technical solutions 1-13, wherein the GIGYF1 is configured to be delivered to a tissue or cell selected from the following group: liver, heart, muscle, pancreas and brain.
15. The use according to any one of technical solutions 1-14, wherein the disease and/or symptom is associated with GIGYF1 expression.
16. The use according to any one of technical solutions 1-15, wherein the GIGYF1 is used to alleviate, prevent and/or treat a metabolic disease and/or symptom.
17. The use according to any one of technical solutions 1-16, wherein the GIGYF1 is used to alleviate, prevent and/or treat diabetes and/or a complication thereof.
18. The use as described in any one of technical solutions 1-17, wherein the disease and/or symptom is selected from the following group: developmental delay, hyperglycemia, hyperlipidemia, increased ratio of glycosylated hemoglobin, insulin resistance, decreased glucose tolerance, increased fat, fatty liver, spleen hyperplasia, decreased insulin secretion, increased glucagon secretion, decreased reproductive capacity, kidney damage, skin damage, swollen finger (toe) joints, heart damage, and cognitive impairment.
19. A cell comprising a formulation for interfering with GIGYF1, GIGYF1 variants, truncates and analogs thereof.
20. A kit comprising a nucleic acid encoding GIGYF1, GIGYF1 variants, truncated versions and analogs thereof, a vector comprising the above nucleic acid and/or the cell according to technical solution 19.

Without being limited by any theory, the following examples are only for illustrating the methods and uses of the present application, etc., and are not intended to limit the scope of the invention of the present application.

### EXAMPLES

### Example 1

The present application utilizes a wild-type gene sequence encoding GIGYF1 protein, which could be optionally optimized. The sequence of the GIGYF1 protein encoded by the *GIGYF1* gene could be as set forth in SEQ ID NO: 1, and the sequence of the *GIGYF1* gene could be as set forth in SEQ ID NO: 5.

Viral vector construction. The gene fragment encoding GIGYF1 of the present application was linked to a plasmid vector to obtain a linked product and a recombinant adeno-associated virus expression vector. Cells containing the virus vector could be obtained by transfecting a host cell with the recombinant adeno-associated virus expression vector. After testing, the titer and purity of the generated virus met the requirements of subsequent applications.

### Example 2

### Animal model construction

A model animal was selected, and the gene of *Gigyf1* was knocked out by gene editing tools. For example, a guide RNA as set forth in SEQ ID NO: 3 or 4 could be provided.

Through PCR detection, sequencing analysis and growth observation, the constructed animal model had good growth ability and had the effect that the gene of *Gigyf1* could not be expressed. Through phenotypic observation and detection, the *Gigyf1* knockout animal model had at least the following effects: (1) developmental delay, such as similar conditions to early dwarfism; (2) abnormal blood indicators, such as high blood lipids, thick blood, etc.; (3) abnormal glucose metabolism, with diabetes and hyperglycemia phenotypes, such as impaired intraperitoneal glucose tolerance, resistance to insulin, abnormal pancreatic islet morphology; (4) abnormal fat metabolism, for example, increased fat mass in females, decreased fat mass in males, fatty liver, lipomas and spleen hyperplasia, etc.; (5) reproductive abnormalities, for example, decreased sperm count in male mice; (6) poor renal function, for example, abnormal urine color (yellow urine); (6) skin complications similar to diabetes, such as partial hair loss, skin ulcers, swollen finger (toe) joints, etc.; (7) increased mortality; (8) abnormal heart condition; (9) cognitive abnormalities.

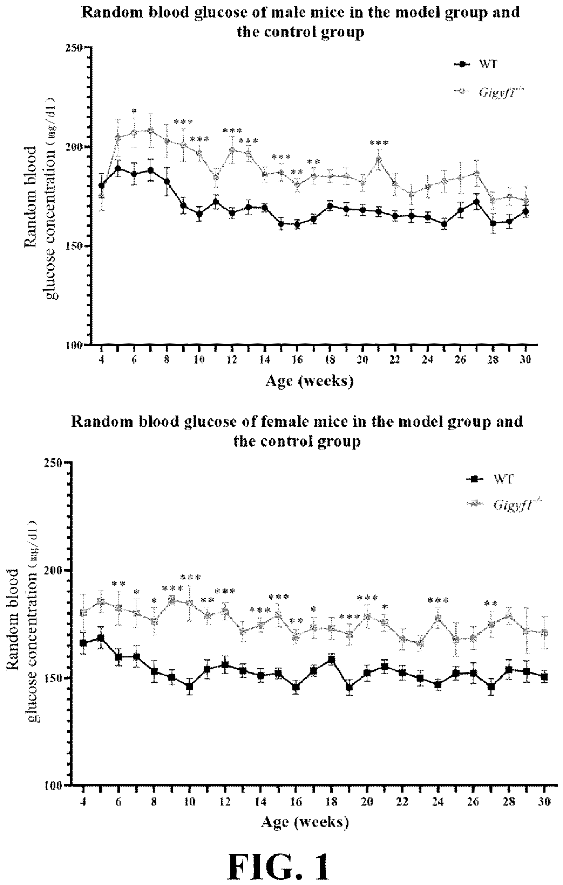

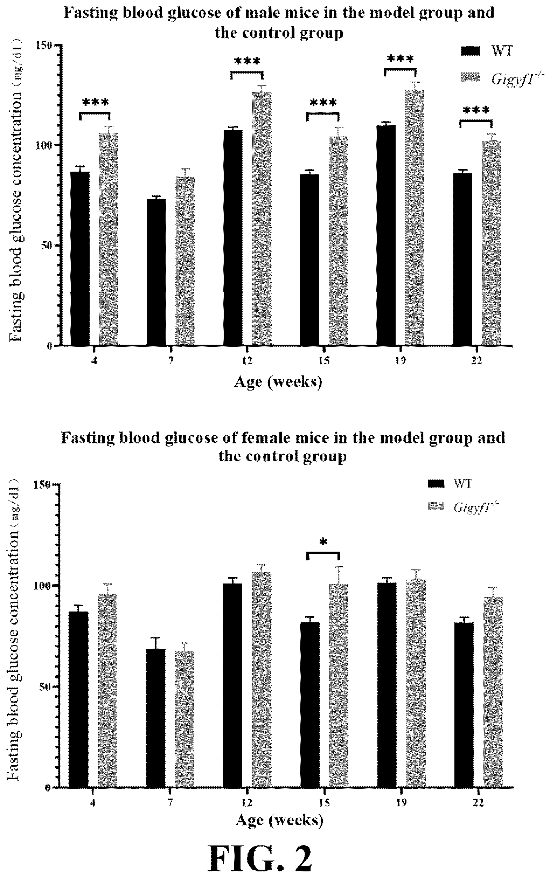

As shown in FIG. 1 and 2, blood glucose levels were measured at the same time each week in wild-type mice (n=40 males, 31 females) and *Gigyf1* knockout mice (n=39 males, 18 females) by the glucose dehydrogenase method. The blood glucose levels of *Gigyf1* knockout mice were abnormal. The blood glucose levels of *Gigyf1* knockout mice were higher than those of wild-type mice both under fed and fasted conditions.

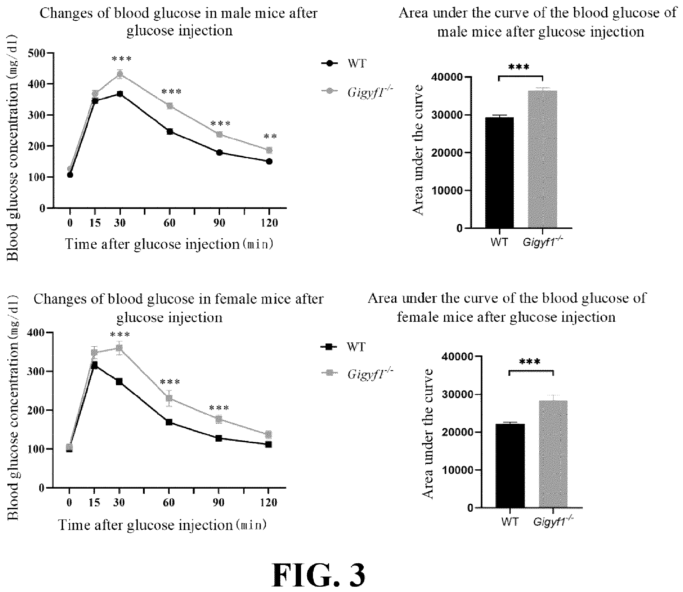

As shown in FIG. 3, mice were fasted for 14-16 hours with free access to water, and then taken out. Blood was collected from the tail vein, and the basal blood glucose value (i.e., blood glucose at 0 min) was detected by the glucose dehydrogenase method. Following body weight measurement, glucose solution was intraperitoneally injected into the mouse at a dose of 2 g glucose/kg body weight, and timing began immediately after the injection. Blood glucose values were recorded at 15 min, 30 min, 60 min, 90 min, and 120 min, respectively. In the IPGTT test, the glucose clearance rate of *Gigyf1* knockout mice (n=30 males, 16 females) was slower than that of wild-type mice (n=37 males, 31 females), indicating that *Gigyf1* knockout mice (males and females) had reduced glucose utilization efficiency and had a diabetic phenotype.

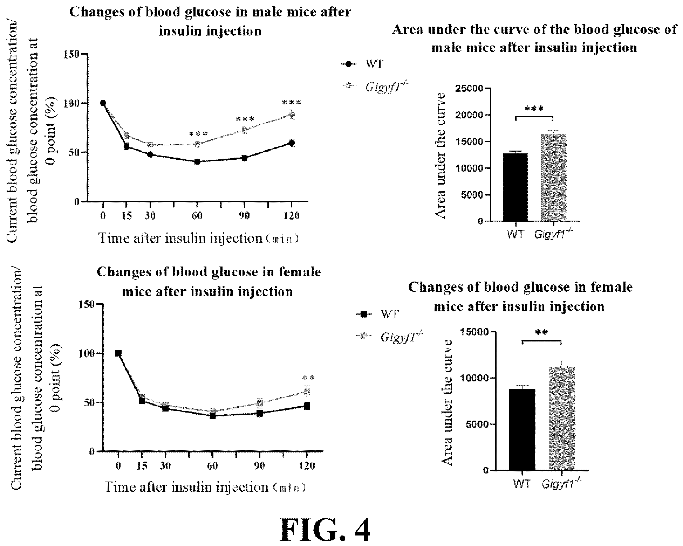

As shown in FIG. 4, the method of blood sampling from the tail vein was also adopted in the ITT test, and the basal blood glucose value (i.e., blood glucose at 0 min) was measured with a blood glucose meter, and the body weight was measured and recorded. 0.1 U/mL insulin-saline solution was prepared and mixed thoroughly. Timing was started immediately after intraperitoneal injection of 1 U/kg body weight and blood glucose values were recorded at 15 min, 30 min, 60 min, and 120 min, respectively. The blood glucose value was normalized to the blood glucose value at 0 min as "1" to obtain the ITT curve and the area under the curve was calculated. *Gigyf1* gene knockout mice (n = 20 males, 11 females) were less sensitive to insulin than wild-type mice (n = 31 males, 18 females).

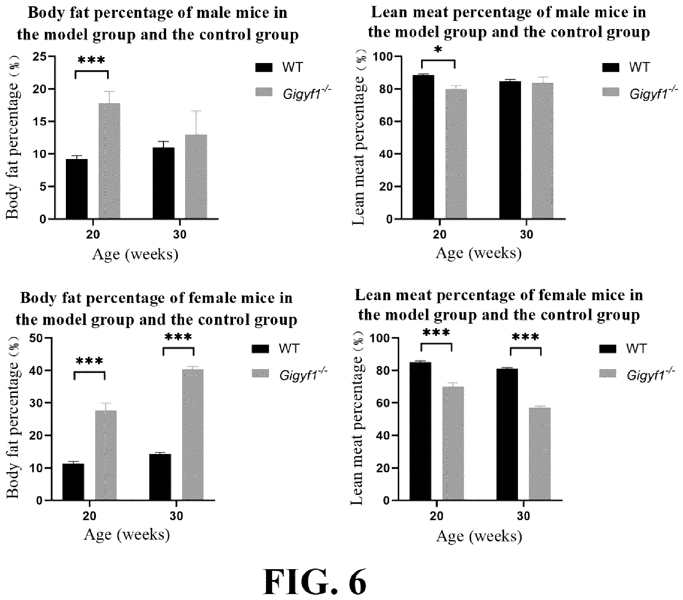

As shown in FIG. 6, after measuring the body weight of wild-type mice (n = 20 males, 18 females) and *Gigyf1* knockout mice (n = 10 males, 10 females), the fat and lean meat of each mouse were analyzed gravimetrically using a nuclear magnetic resonance biopsy analyzer. The body fat percentage and lean meat percentage could be obtained by dividing each component by the body weight. It can be found that the fat proportion of *Gigyf1* knockout female mice was higher than that of wild-type female mice and has been increasing, while the fat proportion of *Gigyf1* knockout male mice was higher than that of wild-type male mice but has been decreasing.

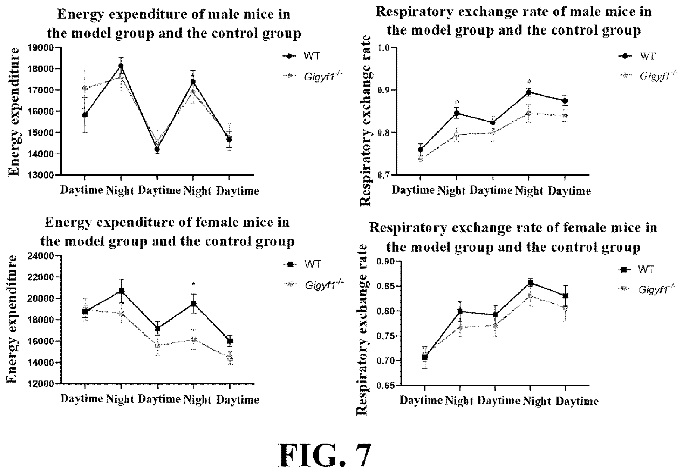

As shown in FIG. 7, wild-type mice (n = 8 males, 6 females) and *Gigyf1* knockout mice (n = 8 males, 6 females) were weighed and placed in metabolic cages at 9 o'clock in the morning. Oxygen consumption, carbon dioxide production and activity were recorded for two days. The energy expenditure and respiratory exchange rate of the mice were calculated. The respiratory exchange rate of the gene knockout female and male mice was significantly lower than that of the wild type, indicating that the gene knockout mice were more inclined to use fat as an energy source for their metabolism than the wild-type mice.

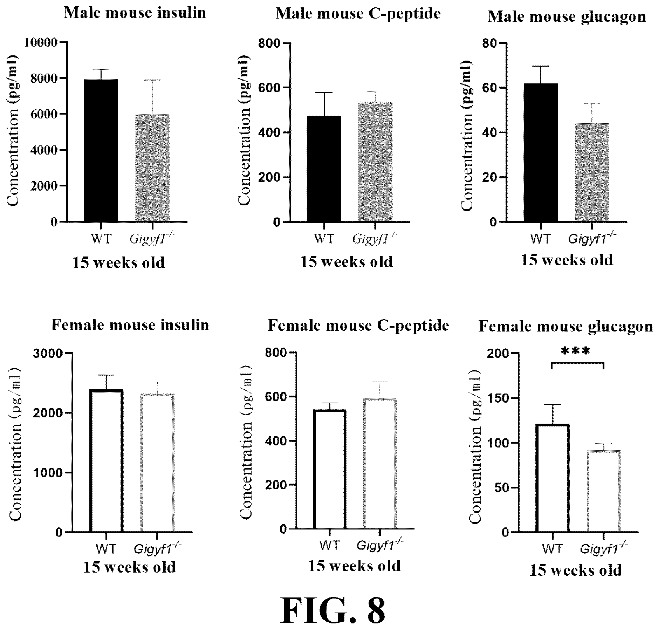

As shown in FIG. 8, when the mice were 15 weeks old, blood was collected from the orbits of wild-type mice (n = 8 males, 6 females) and *Gigyf1* knockout mice (n = 7 males, 7 females) after 16 hours of starvation. The whole blood samples were left at room temperature for 0.5-1 hour and centrifuged at 4°C at 3500 rpm for 10 minutes to obtain serum. The corresponding ELISA kit was used for detection, and then the levels of C-peptide, insulin and glucagon were detected. The serum was centrifuged before use. There were no significant differences in insulin, glucagon, and C-peptide in the serum of KO mice compared with WT mice, indicating no significant changes in pancreatic islet function.

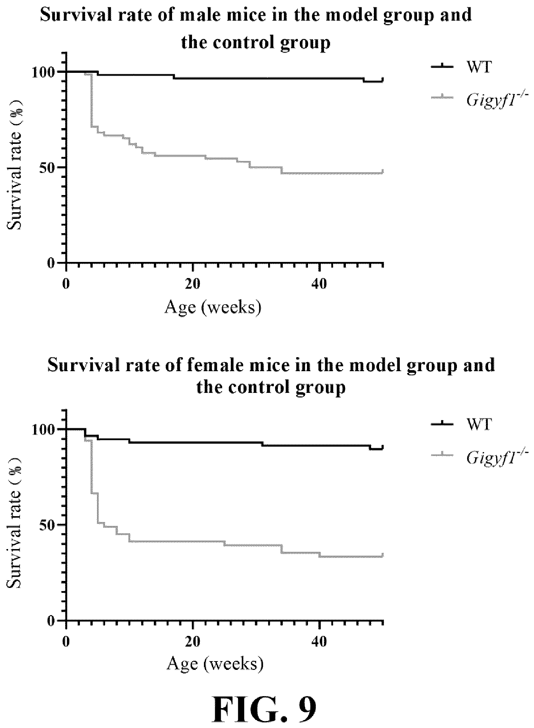

As shown in FIG. 9, the survival of wild-type mice (n = 58 males, 59 females) and *Gigyf1* knockout mice (n = 65 males, 52 females) was recorded for 50 weeks, and the survival curve was plotted accordingly. It can be seen that *Gigyf1* gene deletion would lead to high mortality, with the most pronounced deaths occurring during the weaning period.

### Example 3

The present application provides a recombinant nucleic acid encoding GIGYF1 protein, and the recombinant nucleic acid could be used for the intervention, alleviation and treatment of diseases and/or symptoms caused by abnormalities in the gene encoding GIGYF1 protein.

### Therapeutic effect of recombinant nucleic acid of gigyf1

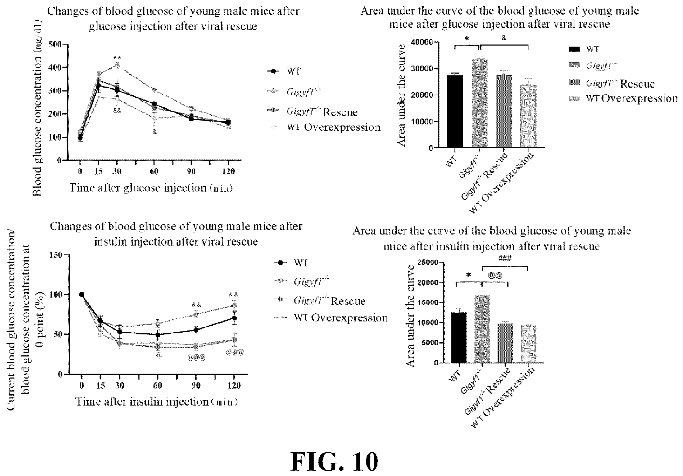

15-week-old wild-type mice (n = 3 males) and *Gigyf1* knockout mice (n = 3 males) were injected intraorbitally with AAV9 virus at a titer of 1*10^14 vg/kg for systemic *GIGYF1* rescue. In the fourth week after the injection, IPGTT and ITT tests were performed on the above two groups and wild-type mice (n = 3 males) and *Gigyf1* knockout mice (n = 3 males) that did not exhibit rescue, as shown in FIG. 10.

During the IPGTT test, mice were fasted for 14-16 hours with free access to water, and then taken out. Blood was collected from the tail vein, and the basal blood glucose value (i.e., blood glucose at 0 min) was detected by the glucose dehydrogenase method. Following body weight measurement, glucose solution was intraperitoneally injected into the mouse at a dose of 2 g glucose/kg body weight, and timing began immediately after the injection. Blood glucose values were recorded at 15 min, 30 min, 60 min, 90 min, and 120 min, respectively. After rescue of *Gigyf1,* the ability of *Gigyf1* knockout mice to clear glucose from the blood was restored to the level of wild-type mice, and the blood sugar at 0 point (starvation blood sugar) also dropped, indicating that the diabetic phenotype was ameliorated after rescue treatment; in addition, overexpression of *GIGYF1* in wild-type mice showed a significant effect on improving the ability to clear glucose, indicating that overexpression of this gene had a preventive effect on diabetes.

The mice in the ITT test were not starved, and the test also adopted the method of blood sampling from the tail vein. The basal blood sugar value (i.e., blood sugar at 0 min) was measured with a blood glucose meter, and the body weight was weighed and recorded. 0.1 U/mL insulin-saline solution was prepared and mixed thoroughly. Timing was started immediately after intraperitoneal injection of 1 U/kg body weight and blood glucose values were recorded at 15 min, 30 min, 60 min, and 120 min, respectively. The blood glucose value was normalized to the blood glucose value at 0 min as "1" to obtain the ITT curve and the area under the curve was calculated. The experimental results showed that after gene therapy, *Gigyf1* knockout mice exhibited a significant increase in insulin sensitivity, and there was a trend towards higher insulin efficiency compared with WT results; overexpression of *GIGYF1* in wild-type mice also demonstrated that overexpression of this gene had a preventive effect on diabetes.

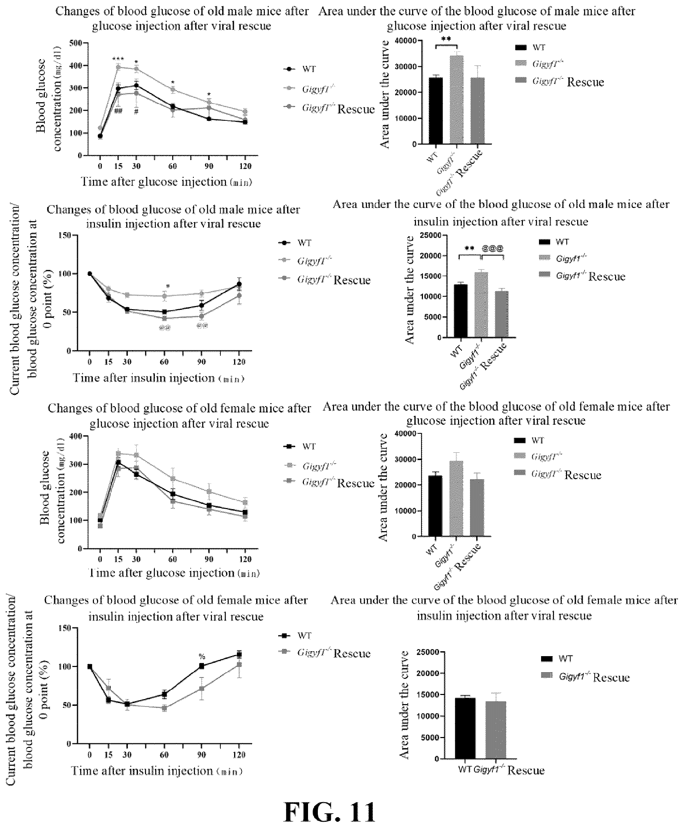

40-week-old *Gigyf1* knockout mice (n = 4 males, 5 females) were injected intraorbitally with AAV9 virus at a titer of 1*10^14 vg/kg for systemic GIGYF1 rescue. In the fourth week after the injection, IPGTT and ITT tests were performed on the above groups exhibiting rescue and wild-type mice (n = 10 males, 7 females) without exhibiting rescue and *Gigyf1* knockout mice (n = 4 males). As shown in FIG. 11, the traits of the gene-rescued *Gigyf1* knockout mice returned to the wild-type level, or even better than the wild-type.

The results showed that gene rescue in both young and old mice, and in both female and male mice, had the effect of treating and preventing diabetes.

The foregoing detailed description is provided by way of explanation and example and is not intended to limit the scope of the appended claims. Various modifications to the embodiments described herein will be apparent to those of ordinary skill in the art and remain within the scope of the appended claims and their equivalents.

## Claims

1. Use of a formulation for interfering with GIGYF1, GIGYF1 variants, truncated versions and analogs thereof in the preparation of a drug for alleviating, preventing and/or treating a specific disease and/or symptom.

2. The use according to claim 1, wherein the GIGYF1 comprises an amino acid sequence as set forth in SEQ ID NO: 2.

3. The use according to claim 1, wherein the GIGYF1 comprises an amino acid sequence as set forth in SEQ ID NO: 1.

4. The use according to any one of claims 1-3, wherein a nucleotide sequence encoding the GIGYF1 is as set forth in SEQ ID NO: 5.

5. The use according to any one of claims 1-4, wherein the GIGYF1 is configured to be delivered to a tissue or cell selected from the following group: liver, heart, muscle, pancreas and brain.

6. The use according to any one of claims 1-5, wherein the disease and/or symptom is associated with GIGYF1 expression.

7. The use according to any one of claims 1-6, wherein the GIGYF1 is used to alleviate, prevent and/or treat a metabolic disease and/or symptom.

8. The use according to any one of claims 1-7, wherein the GIGYF1 is used to alleviate, prevent and/or treat diabetes and/or a complication thereof.

9. The use according to any one of claims 1-8, wherein the disease and/or symptom is selected from the following group: developmental delay, hyperglycemia, hyperlipidemia, increased ratio of glycosylated hemoglobin, insulin resistance, decreased glucose tolerance, increased fat, fatty liver, spleen hyperplasia, decreased insulin secretion, increased glucagon secretion, decreased reproductive capacity, kidney damage, skin damage, swollen finger (toe) joints, heart damage, and cognitive impairment.

10. A cell comprising a formulation for interfering with GIGYF1, GIGYF1 variants, truncates and analogs thereof.

11. A kit comprising a nucleic acid encoding GIGYF1, GIGYF1 variants, truncated versions and analogs thereof, a vector comprising the above nucleic acid and/or the cell according to claim 10.

12. A modified non-human animal, wherein the expression level of GIGYF1 gene of the modified non-human animal is reduced compared with a wild-type non-human animal.

13. The modified non-human animal according to claim 12, wherein the GIGYF1 gene is knocked out or knocked down.

14. The modified non-human animal according to claim 12, wherein the GIGYF1 gene is knocked out by CRISPR/Cas technology.

15. The modified non-human animal according to claim 12, wherein the modified non-human animal exhibits a phenotype and/or symptom of a metabolic disease.

16. Use of the non-human animal according to any one of claims 12-15 in screening a drug, wherein the drug is used to alleviate, prevent and/or treat a metabolic disease and/or symptom.

17. The use according to claim 16, wherein the drug is used to alleviate, prevent and/or treat diabetes and/or a complication thereof.
